# EUROPEAN PATENT APPLICATION

(11) **EP 1 304 111 A2**
(43) Date of publication of application: **23.04.2003**
(21) Application number: 02257249.9
(22) Date of filing: 18.10.2002
(51) Int. Cl.: A61K 31/549, A61P 35/00

(54) **Use of methylol-containing compounds for the treatment of breast cancer**

(30) Priority: 19.10.2001 US 330082 P
(71) Applicant: ED. GEISTLICH SÖHNE AG FÜR CHEMISCHE INDUSTRIE, 6110 Wolhusen (CH)
(72) Inventor: Redmond, Paul H., Wilton, Cork (IE); Pfirrmann, Rolf, W., 6006 Lucerne (CH)
(74) Representative: Marsden, John Christopher

(57) **Abstract**

Methylol-containing compounds such as taurolidine, taurultam and mixtures thereof are useful in the manufacture of medicaments for treatment or prophylaxis of breast cancer tumours and metastases in mammalian subjects.

## Description

### Related Application Data

This application claims the benefit of United States provisional application 60/330,082 filed October 19, 2001.

### Technical Field

The invention relates to the treatment of breast cancer.

### Background of the Invention

Breast cancer treatment typically involves removal of the tumor and/or breast, sometimes along with associated surrounding tissue, followed by chemotherapy and/or radiation therapy. However, there remains a need in the art for improved methods and treatment for combatting breast cancer.

### SUMMARY OF THE INVENTION

In accordance with the present invention, a method of treating a patient with breast cancer comprises administering to the patient a breast cancer cell proliferation-inhibiting amount of a methylol-containing compound.

### DETAILED DESCRIPTION OF THE PREFERRED EMBODIMENTS

A method of treating breast cancer is provided, whereby apoptotic and/or necrotic death of a primary breast cancer cell, or metastases thereof, is brought about by contacting the cell with a proliferation-inhibiting amount of a methylol-containing compound.

Methylol transfer agents include taurolidine and the related compound taurultam. Taurolidine acts by transferring three methylol groups at the site of action, taurultam being an intermediate metabolite which itself transfers a single methylol group with liberation of the very well tolerated compound taurinamide. Thus, the two compounds act by essentially the same mechanism. It should be noted that methylol transfer is to be contrasted with methyl transfer which is characteristic of many highly toxic anti-tumor drugs. Taurolidine and taurultam have low toxicity and are not cytotoxic against normal cells.

One embodiment involves treatment of a patient with breast cancer by administering to the patient a breast cancer cell proliferation-inhibiting amount of a methylol-containing compound.

One embodiment includes administering a methylol-containing compound to the patient after surgical removal of a breast cancer tumor from the patient. In another embodiment, the methylol-containing compound is further administered to the patient prior to surgical removal of the tumor from the patient, as well as thereafter.

One embodiment comprises administration of a methylol transfer agent in at least two dosing cycles, each cycle comprising an administration phase and a non-administration (rest) phase, the administration phase comprising administration, preferably by infusion, of a daily dose of the methylol transfer agent for about 1 to 8 days, followed by a non-administration (rest) phase of about 1 to 14 days during which no methylol transfer agent is administered.

Preferred methylol transfer agents are taurolidine, taurultam, and mixtures thereof.

The present method is carried out by administering, to a patient with breast cancer, compositions containing an active methylol-containing compound, at a dose sufficient to inhibit proliferation of breast cancer cells. By "methylol-containing compound," or "methylol transfer agent," is meant a compound which contains or is capable of producing a methylol molecule under physiological conditions. A methylol-containing compound is characterized as having a R-CH₂-OH group in which R is an alkyl, aryl or hetero group. The invention also includes the use of compounds capable of producing or being converted into a compound containing a R-CH₂-OH structure.

Methylol transfer agents include methylol-containing compounds such as taurolidine and taurultam, and their derivatives. The compounds taurolidine and taurultam are disclosed in U.S. Patent No. 5,210,083. Other suitable methylol-containing compounds include taurinamide derivatives and urea derivatives. Examples of derivatives of taurolidine, taurultam, taurinamide and urea which may be useful in the present invention can be found in WO 01/39763A2. Particularly preferred methylol transfer agents for utilization in accordance with the present invention are taurolidine, taurultam, biologically active derivatives thereof and mixtures thereof.

Other methylol-containing compounds suitable for inducing apoptotic and/or necrotic death of cancer cells include but are not limited to 1,3,-dimethylol-5,5-dimethylhydantoin, hexamethylene tetramine, or noxythiolin. By derivative of taurolidine or taurultam is meant a sulfonamide compound which possesses at least 10% of the neoplastic activity of taurolidine or taurultam, respectively. A sulfonamide compound is one having a R₂N-SO₂R' formula. Derivatives of the compounds described herein may differ structurally from a reference compound, e.g., taurolidine or taurultam, but preferably retain at least 50% of the biological activity, e.g., induction of tumor cell death, of the reference compound. Preferably, a derivative has at least 75%, 85%, 95%, 99% or 100% of the biological activity of the reference compound. In some cases, the biological activity of the derivative may exceed the level of activity of the reference compound. Derivatives may also possess characteristics or activities not possessed by the reference compound. For example, a derivative may have reduced toxicity, prolonged clinical half-life, etc.

The invention includes administration of a methylol-containing compound such as taurolidine and/or taurultam, for the treatment or prophylaxis of breast cancer tumors and metastases in mammalian subjects.

It is particularly beneficial for administration of methylol-containing compound such as taurolidine and/or taurultam to prevent the spread of metastases, especially following surgical removal of tumors from a human breast and/or associated tissue.

The invention further includes the use of a methylol-containing compound such as taurolidine and/or taurultam, for the preparation of pharmaceutical compositions for the treatment or prophylaxis of breast cancer tumors and metastases in mammalian subjects.

Effective dosage amounts of a methylol transfer agent in accordance with the present invention may comprise pharmaceutical dosage units within the range of about 0.1-1,000 mg/kg, preferably 150-600 mg/kg per day, and most preferably 300-450 mg/kg per day. Alternatively, the dosages can be administered on a grams/day basis, from about 1-100 g/day, e.g., from about 2-60 g/day. Preferred doses may be in the range of about 2.5-30 g/day taurolidine, 4-60 g/day taurultam, or a mixture thereof. Most preferred doses are in the range of about 10-20 g/day taurolidine, 20-40 g/day taurultam, or a mixture thereof.

Suitable formulations for injection or infusion may comprise an isotonic solution containing one or more solubilizing agents, e.g., polyols such as glucose, in order to provide solutions of increased taurolidine or taurultam concentration. Such solutions are described in EP 253662B1. The concentration of taurolidine or taurultam in such solutions may be in the range 1-60 g/liter.

Methylol transfer agents are generally poorly soluble in water. Thus, it is often required to administer relatively large volumes of aqueous solutions containing taurolidine or taurultam, for example 10g to 30g of taurolidine and/or taurultam. Preferred solutions for administration in accordance with the present invention contain from about 0.5-2% taurolidine and/or taurultam. It may be convenient to administer these compounds by infusion in view of the relatively large volumes concerned, conveniently at intervals throughout the day.

Administration, preferably by infusion, of the total daily dose can be carried out at a consistent rate over 24 hours, or according to a more rapid infusion schedule of the dose in portions, with breaks between each portion of the dose, e.g. infusion of 250 ml of a 2% taurolidine solution (5 g dose) over 2 hours, followed by a brief break of 4 hours, repeated over the course of a 24 hour infusion period to achieve a total daily dose of 20 g. Alternatively, 250 ml of a 2% taurolidine solution may be infused over one hour, with a one hour break between dose portions, and repeated until the daily dose is achieved, such that the total daily dose is provided over the course of less than 24 hours (i.e., approximately half the day), with no infusion occurring during the remainder of the day.

In accordance with one embodiment, four bottles (250 ml each) of 2% taurolidine solution are administered intravenously to a breast cancer patient, at a rate of 40 drops per minute, one bottle every six hours. The therapy cycle consists of an administration phase of daily infusions for one week, followed by a rest phase of two weeks. Total treatment comprises at least two such cycles. Taurolidine 2% solution may be administered intravenously, with 25-28 bottles of 250 ml taurolidine 2% solution being instilled per cycle.

In accordance with a second embodiment of the invention, the administration phase comprises a daily regimen whereby 250 ml of taurolidine 2% solution is administered over the course of 2 hours, followed by a four hour break, repeated over 24 hours to achieve the total daily dose.

In accordance with a third embodiment of the invention, the administration phase comprises a daily regimen whereby 250 ml of 2% taurolidine solution is infused over one hour, followed by a one-hour break, and repeated until the daily dose is achieved. If the total dose is 20 g (for example), this regimen would provide the daily dose with four 250 ml infusions of 2% taurolidine over a 7 hour time span. No infusion occurs for the remainder of the day. Infusion rates can be lengthened (e.g., to 250 ml over 90 or 120 minutes), if desired.

In a further embodiment, concomitant administration of anti-convulsants and/or anti-oedema therapy and/or antibiotics and/or fluid and electrolyte replacement is carried out.

### 1. Anti-Convulsants

Preferably, the patient should be stabilized on anti-convulsive medications prior to treatment, to avoid complications during the treatment. This can conveniently be administered in part on an out-patient basis, as well as to prevent any emergency stabilization on an undesired medication. Valproinic acid is the agent of first choice; the dose should be determined in accordance with blood level checks and administered in 2 single doses. Normally, a dose of 1200 mg to 1500 mg is required. If a treatment with valproinic acid is not sufficient, a combination treatment with lamotrigin is possible. In case of allergies or if valproinic acid is not tolerated, the primary stabilization is to be done with lamotrigin. Phenytoin and carbamazepin are contra-indicated.

### 2. Anti-Oedema Therapy

An anti-oedema therapy may also be administered, but only if absolutely necessary, because otherwise focal neurological symptoms may occur or become intensified, or intracerebral pressure may symptoms develop. Dexamethason should be given before or after the taurolidine was administered. The anti-oedema therapy should be administered with dexamethason, using the lowest possible dose. To protect the stomach a concomitant therapy with ranitidine 1 x 150 mg/day may be given. If stomach problems are observed with this therapy, an alternative treatment with antra 1-2 x 20 mg/day should be administered.

In cases of massively elevated intracerebral pressure and insufficient effectiveness of dexamethason, a therapy with mannitol, in particular at a dosage of up to 4 x 250 ml/day, is possible.

### 3. Antibiotic Therapy

A calculated antibiotic treatment with one of the subsequently listed antibiotics may be given, until the arrival of the sensitivity test.
* Urinary tract infection:
   primary: Cotrimoxazol
   alternative: Doxycyclin
* Pneumonia:
   primary: Erythromycin
   alternative: Doxycyclin
   The following antibiotics should only be used if absolutely necessary (in the most severe, life-threatening infections) and if the sensitivity situation warrants it: Chino lone, penicillin, cephalosporin

### 4. Fluid and Electrolyte Replacement in Connection with Intravenous Taurolidine 2% Therapy

An amount of 250 ml of full electrolyte solution is preferably be given at the same time and with the same infusion speed parallel to the infusion with 250 ml taurolidine 2%. Electrolytes and blood count should be monitored twice per day, and the central vein pressure should be checked once daily.

If a hypernatraemia is observed, first, it should be determined whether dehydration is the cause. Diuretic agents should only be used if fluid is replaced at the same time and after dehydration was ruled out as the reason.

The methylol-containing compound is administered alone or in combination with one or more additional antineoplastic agents. For example, an antimetabolite, a purine or pyrimidine analogue, an alkylating agent, crosslinking agent (e.g., a platinum compound), and intercalating agent, and/or an antibiotic is administered in a combination therapy regimen. The supplemental drug is given before, after, or simultaneously with the methylol-containing agent.

The invention also includes treating a drug resistant tumor, e.g., a multiple drug resistant (MDR) tumor, in a mammal by administering to the mammal a methylol-containing compound.

According to another embodiment, a solution containing taurolidine and/or taurultam further contains taurin, in an amount within a range of about 1-20 g/l, preferably about 5 g/l.

The invention is illustrated by the following examples, which are not intended to be limiting.

### Example 1: Hypotonic Solution 2% Taurolidine

One suitable composition for intravenous drop infusion is shown below. Isotonic sterile solution, 100 ml:

| | |
|---|---|
| 2.0 g | Taurolidine |
| 5.0 g | PVP 16 PF UP aqua dest. ad solut. 100 ml. PH 7.2 - 7.3 |
| Sterile-filtered and steam sterilization. | |

### Example 2: Isotonic Solution 2% Taurolidine with Taurin and electrolytes

Another suitable composition for intravenous drop infusion is shown below. Isotonic sterile solution, 100 ml:

| | |
|---|---|
| 2.0 g | Taurolidine |
| 5.0 g | PVP 17 PF UP |
| 0.5 g | Taurin |
| 0.3 g | Sodium chloride |
| Sterile-filtered and steam sterilization | |

### Example 3: Isotonic Ringer Solution 2% Taurolidine with Taurin and electrolytes

Another suitable composition for intravenous drop infusion is shown below. Isotonic sterile solution, 100 ml:

| | |
|---|---|
| 2.0 g | Taurolidine |
| 5.0 g | PVP 17 PF UP |
| 0.5 g | Taurin |
| 0.26 g | Sodium chloride |
| 0.0033 | g Potassium chloride |
| 0.004 g | Calcium chloride 2H₂O |
| 0.003 g | Sodium hydrogen carbonate |
| Sterile-filtered and steam sterilization | |

### Example 4: Ringer-Lactate 2% Taurolidine with Taurin and electrolytes

Another suitable composition for intravenous drop infusion is shown below. Isotonic sterile solution, 100 ml:

| | |
|---|---|
| 2.0 g | Taurolidine |
| 5.0 g | PVP 17 PF UP |
| 0.5 g | Taurin |
| 0.20 g | Sodium chloride |
| 0.013 | g Potassium chloride |
| 0.009 | g Calcium chloride 2H₂O |
| 0.0033 | g Sodium lactate 50% solution (Pharmacopeia Europea) |
| Sterile-filtered and steam sterilization | |

### Example 5: 3% or 2% Taurultam drop infusions solutions

One preferred solution comprises:

| | |
|---|---|
| 3.0 g or 2.0 g | Taurultam |
| 5.0 g | PVP 16 PF UP |
| aqua dest. ad solution 100 ml, | |
| bottles of 250 ml or 500 ml | |

### Example 6: Isotonic 2% Taurultam drop infusions solution

| | |
|---|---|
| 2.0 g | Taurultam |
| 5.0 g | PVP 16 PF UP |
| 0.5 g | NaCI |
| 0.005 g | KCI |
| 0.0066 g | CaCl₂ |
| 0.005 g | NaHCO₃ |
| aqua dest. ad solution 100 ml, | |
| bottles of 250 ml or 500 ml | |

### Example 7: Taurolidine Attenuates Primary and Metastatic Tumor Burden in an Orthotopic Breast Cancer Model

Female BALB/c mice were inoculated with 5 x 10⁴ murine 4T1 breast carcinoma cells by mammary fat pad injection. Alternate day tail vein injection of 400µl 2% Taurolidine or 400µl 5% PVP (vehicle) was commenced on day 12. Animals were sacrificed on day 20 at which time final tumor volume and wet lung/body weight ratio were assessed. Cell proliferation and apoptosis were evaluated using haematoxylin and eosin histochemistry.

Taurolidine significantly reduced primary tumor volume, and significantly dowregulated tumor cell proliferation while enhancing apoptosis. Lung/body weight ratio was also significantly reduced following taurolidine therapy (table 1).

**Table 1:**

| * p<0.05 significant, t-test | | | |
|---|---|---|---|
| | Primary Tumor | | Lung/body |
| | Vol. cm³ at Day 20 | Mitosis/Apoptosis ratio | Wt. ratio |
| PVP | 1.859 +/-0.60 | 1.187 +/-0.201 | 901 +/-140 |
| Taurolidine | 0.914 +/-0.14 * | 0.390 +/-0.122* | 658 +/-71 * |

Taurolidine therapy led to significant reductions in both primary and metastatic tumor burden. The anti-neoplastic properties of taurolidine may relate to its ability to regulate cell cycle as well as apoptotic events. These findings demonstrate the efficacy of taurolidine treatment in a murine model of breast cancer.

### Example 8: Taurolidine inhibits breast cancer in vitro and in vivo

The metastatic human breast cell line MDA and the murine 4T1 breast cell line were incubated with taurolidine at 5, 10, 25, 50 and 100 µg/ml. Cell proliferation was assessed using BrdU incorporation and apoptosis was assessed using a propidium iodide solution. BALB/c mice received i.v. tail vein injection of 200µl 4T1 (5 x 10⁵/ml), and a subsequent injection of 400µl of taurolidine or phosphate buffered saline (PBS) every second day, up to day 9. The animals were sacrificed on day 10 and the following assessments were performed: (a) gross metastatic score and (b) histological assessment by a grid mechanism. A p-value of <5% was taken as statistically significant.

Taurolidine significantly inhibited tumor cell proliferation at 24 hours at the 50 and 100µg/ml dosages by approximately 60% and 90% respectively (p<0.0001). This was due to a direct cytotoxic effect on the cells. The gross metastatic score was 1.89 in the taurolidine group vs. 3.4 in the PBS group (p=0.05). The percentage of metastases in the lung tissue was 4.5% in the taurolidine group vs. 11.4% in the PBS group.

Taurolidine inhibits the proliferation of these breast cancer cell lines *in vitro* at sub-therapeutic dosages. Taurolidine also significantly decreased the number of lung metastases in this model. These findings show efficacy of taurolidine in treating breast cancer.

### Example 9: Intra-Venous Taurolidine Confers a Survival Benefit Following Surgical Resection of Primary Breast Tumor Mass in a Murine Model

The effect of i.v. taurolidine was examined on metastic tumor burden and overall survival, following primary tumor resection in an orthotopic breast cancer model.

Female BALB/c mice were inoculated with 5 x 10⁴ murine 4T1 breast carcinoma cells by mammary fat pad injection (n=20). Primary tumors were resected on day 12 at which time alternate day tail vein injection of 400µl 2% Taurolidine or 400µl 5% PVP (vehicle) was commenced. Animals were sacrificed on day 30 at which time wet lung/body weight ratio was assessed. A survival study was also performed with n=5 mice per treatment group.

Lung/body weight ratio was significantly reduced following taurolidine therapy (Table 2). Intra-venous taurolidine significantly improved survival following primary tumor resection (p=0.001, Log Rank).

**Table 2**

| *p=0.015 significant, t-test | |
|---|---|
| | Lung/body Wt. ratio |
| PVP | 1250 +/- 297 |
| Taurolidine | 965 +/- 157 * |

Taurolidine therapy led to significant reductions in metastatic tumor burden, resulting in a significant improvement in survival time.

### Example 10: Two-cycle dosing Schedule for Treating Patients with Breast Cancer Using Intravenous Taurolidine 2%

Four bottles (250 ml each) of 2% taurolidine solution are administered intravenously to patients with cancer, at a rate of 40 drops per minute, one bottle every six hours. The dosing cycle includes an administration phase of daily infusions for one week, followed by a non-administration phase of two weeks, then followed by another administration phase of four bottles per day as previously indicated. Taurolidine 2% solution may be administered intravenously with 25-28 bottles of 250 ml taurolidine 2% solution being instilled per cycle.

### Example 11: Four-cycle Dosing Schedule for Treating Patients with Breast Cancer Using Intravenous Taurolidine 2%

The treatment comprises a minimum of 4 cycles. Each cycle is 7 days long, and is comprised as follows:

### 1. First Cycle

a. Intravenous infusion of 250 ml taurolidine 2% and 250 ml full electrolyte solution via the central vein catheter with an infusion time of 60 minutes.
b. If this therapy causes an elevated liver count, it is necessary to increase the infusion time to 90 or 120 minutes.
c. 60-minute break
d. Repeat the therapies under a or b and c for a total of 6 times per day.
e. At an infusion time of 60 minutes the duration of the daily infusion program per 250 ml of taurolidine is 11 hours, at 90 minutes of infusion time 14 hours, and at 120 minutes of infusion time 17 hours. No drug is administered for the remainder of the time.
f. rest phase

### 2. Subsequent Cycles

a. Intravenous infusion of 250 ml taurolidine 2% and 250 ml full electrolyte solution via the central vein catheter with an infusion time of 60 minutes.
b. If this therapy causes an elevated liver count, it is necessary to increase the infusion time to 90 or 120 minutes.
c. 60 minute break
d. Repeat the therapies under a or b and c for a total of 4 times per day.
e. At an infusion time of 60 minutes the duration of the daily infusion program per 250 ml of taurolidine is 7 hours, at 90 minutes of infusion time 9 hours, and at 120 minutes of infusion time 11 hours. No drug is administered for the remainder of the time.

## Claims

1. Use of a methylol-containing compound in the preparation of a pharmaceutical composition for treatment or prophylaxis of breast cancers and/or metastases in a mammalian subject.

2. Use as claimed in claim 1 wherein said methylol-containing compound is taurolidine, tuarultam or a mixture thereof.

3. Use as claimed in claim 1 or claim 2 wherein said composition is formulated for administration at a daily dosage in the range 1-100 g.

4. Use as claimed in claim 3 wherein said composition is formulated for administration at a daily dosage in the range 2-60 g.

5. Use as claimed in claim 4 wherein said composition is formulated for administration at a daily dosage in the range 4-30 g.

6. Use as claimed in claim 1 or claim 2 wherein said composition is formulated for administration at a daily dosage in the range 150-600 mg/kg.

7. Use as claimed in claim 6 wherein said composition is formulated for administration at a daily dosage in the range 300-450 mg/kg.

8. Use as claimed in any of the preceding claims wherein said composition is formulated for intravenous administration.

9. Use as claimed in any of the preceding claims wherein said composition is formulated for administration during each day of a dosing cycle comprising a plurality of days.

10. Use as claimed in any of claims 1 to 8 wherein said composition is formulated for administration at a total daily dosage of 2-60 g during two dosing cycles of 1-8 days, said dosing cycles being separated by a non-administration phase of 1-14 days.

11. Use as claimed in any of claims 8 to 10 wherein said composition is formulated for continuous infusion over 24 hours.

12. Use as claimed in any of claims 8 to 10 wherein said composition is formulated for infusion of the daily dosage as a series of partial doses whereby each partial dose infusion may be separated by a break during which no infusion occurs.

13. Use as claimed in any of the preceding claims wherein said composition is presented with instructions for administration to said subject after surgical removal of a breast cancer tumour.

14. Use as claimed in any of claims 1 to 12 wherein said composition is presented with instructions for administration to said subject both prior to and after surgical removal of a breast cancer tumour.

15. A pack comprising a methylol-containing compound together with instructions for administration of said compound to a mammalian subject for purposes of treatment or prophylaxis of breast cancers and/or metastases.
